(19) Europäisches Patentamt
European Patent Office
Office européen des brevets

(11) **EP 3 772 492 A1**

(12) **EUROPÄISCHE PATENTANMELDUNG**

(43) Veröffentlichungstag:
**10.02.2021 Patentblatt 2021/06**

(21) Anmeldenummer: **19191036.3**

(22) Anmeldetag: **09.08.2019**

(51) Int Cl.:
*C03C 10/00* (2006.01)   *C03C 3/095* (2006.01)
*C03C 14/00* (2006.01)   *A61K 6/15* (2020.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **VITA-ZAHNFABRIK H. Rauter GmbH & Co. KG**
**79713 Bad Säckingen (DE)**

(72) Erfinder:
• **Goediker, Berit**
  **79713 Bad Säckingen (DE)**
• **Goediker, Michael**
  **79713 Bad Säckingen (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **ROHLING AUS PORÖSER LITHIUMSILIKATGLASKERAMIK MIT FÜLLSTOFF**

(57)  Die vorliegende Erfindung betrifft einen Rohling bestehend aus einer porösen Lithiumsilikatglaskeramik und einem Füllstoff, ein Verfahren zu dessen Herstellung sowie dessen Verwendung als dentale Restauration.

Figur 3

EP 3 772 492 A1

Printed by Jouve, 75001 PARIS (FR)

**Beschreibung**

Die vorliegende Erfindung betrifft einen Rohling bestehend aus einer porösen Lithiumsilikatglaskeramik und einem Füllstoff, ein Verfahren zu dessen Herstellung sowie dessen Verwendung als dentale Restauration.

Unter Restauration versteht man in der Zahnmedizin den Ersatz von Zahndefekten und zerstörtem Zahnersatz durch künstliche Materialien. Die dentalen Restaurationen können dabei einzelne Zähne sowie Gruppen von Zähnen abbilden. Neben der erforderlichen Festigkeit um den während des Kauens auf die Zähne wirkenden Kräften standzuhalten, müssen die Restaurationen darüber hinaus geeignete optische Eigenschaften aufweisen, die es erlauben, die Restaurationen ästhetisch in das bestehende Zahnschema eines Patienten einzupassen.

Dem Fachmann sind eine Reihe von Materialien bekannt, die für die Herstellung dentaler Restaurationen eingesetzt werden können, wobei der Fokus insbesondere auf Keramiken und Glaskeramiken liegt, die sich durch eine hohe Festigkeit auszeichnen. Diese hohe Festigkeit bringt allerdings den Nachteil mit sich, dass sich das Material nur schwer bearbeiten lässt und die Herstellung insbesondere größerer Restaurationen wie Brücken viel handwerkliches Geschick und zahntechnisches Equipment erfordert.

Um die Anforderungen an eine gute Bearbeitbarkeit des Materials sowie eine entsprechende Festigkeit zu erfüllen, wurden im Stand der Technik verschiedene Materialien und Methoden für die Herstellung dentaler Restaurationen vorgeschlagen.

WO 2014/177659 beschreibt einen Rohling zur Herstellung eines dentalen Formteils wie Inlay, Onlay, Krone oder Brücke, wobei der Rohling einen Anteil von mehr als 10 Vol.-% an Lithiumsilikatkristallen enthält und aus einer Glaskeramik mit einer Dichte zwischen 30 und 60% der theoretischen Dichte und aus Glaskeramikpulverpartikeln einer Korngrößenverteilung $d_{90} \leq 80 \ \mu m$ besteht, wobei der Anteil an Lithiumsilikatkristallen 10 Vol.-% bis 90 Vol.-% beträgt. Mit dem beschriebenen Rohling soll es möglich sein, problemlos ein dentales Formteil herauszuarbeiten, wobei das Bearbeiten bei geringem Verschleiß der Werkzeuge möglich sein soll und die aus dem Rohling hergestellten dentalen Formteile sich durch gute mechanische Eigenschaften auszeichnen.

WO 2013/167723 betrifft vorgesinterte Rohlinge für dentale Zwecke auf Basis von Lithiumdisilikat-Glaskeramik, wobei die Rohlinge eine relative Dichte von 60 bis 90%, bezogen auf die Reindichte der Glaskeramik aufweisen. Diese Rohlinge sollen einfach mittels üblicher Fräs- und Schleifverfahren zu Dentalrestaurationen mit der gewünschten Geometrie geformt werden können.

EP 2 269 960 beschreibt ein Verfahren zur Herstellung einer Dentalrestauration, bei dem eine Schmelze eines Ausgangsglases gebildet wird, das die Ausgangskomponenten $SiO_2$, $Li_2O$, $K_2O$, $Al_2O_3$ und $P_2O_5$ als Hauptkomponenten enthält, die Schmelze des Ausgangsglases in eine Form gegossen wird, um einen Ausgangsglasrohling zu bilden und der Glasrohling auf Raumtemperatur abgekühlt wird, der Ausgangsglasrohling eine ersten Wärmebehandlung bei einer ersten Temperatur unterworfen wird, um ein Glasprodukt zu ergeben, das zur Bildung von Lithiummetasilikatkristallen geeignete Keime enthält, oder die Schmelze des Ausgangsglases in eine Form gegossen wird, um einen Ausgangsglasrohling zu bilden und der Rohling auf einer erste Temperatur von 450 bis 550 °C abgekühlt wird, der Ausgangsglasrohling für eine Dauer von 5 bis 50 Minuten bei der ersten Temperatur gehalten wird, um ein Glasprodukt zu ergeben, das zur Bildung von Lithiummetasilikatkristallen geeignete Keime enthält, das Glasprodukt einer zweiten Wärmebehandlung bei einer zweiten Temperatur unterworfen wird, die höher ist als die erste Temperatur, um einen Lithiumsilikatrohling mit Lithiummetasilikatkristallen als Hauptkristallphase zu erhalten und der Lithiumsilikatrohling mit Lithiummetasilikat als Hauptkristallphase mittels maschineller Bearbeitung oder Heißpressen zu einer gewünschten Geometrie geformt wird. Der Rohling soll sich einfach bearbeiten lassen und durch weitere Wärmebehandlungen in ein festes Dentalprodukt überführbar sein.

Um die Bearbeitbarkeit zu verbessern, wurden sogenannte Hybridkeramiken vorgeschlagen, also Keramiken, die mit einem organischem Füllstoff, in der Regel einem Polymer, gefüllt sind.

WO 02/076907 beschreibt einen Verbundwerkstoff mit einer porösen anorganischen nichtmetallischen Matrix und einem zweiten Material, wobei die poröse anorganische nichtmetallische Matrix eine Biegebruchfestigkeit von $\geq 40$ MPa, gemessen nach ISO 6872, besitzt, das zweite Material ein organisches Material ist, das die Poren der porösen Matrix mindestens teilweise ausfüllt und der Verbundwerkstoff einen Elastizitätsmodul $E \geq 25$ GPa, gemessen nach ISO 10477 aufweist.

US 2011/0229858 beschreibt ein Verfahren zur Herstellung eines Verbundblocks für die Herstellung von dentalen Prothesen, wobei ein poröser Träger mit einem flüssigen Harz infiltriert und das Harz anschließend ausgehärtet wird, wobei das flüssige Harz einem Druck von mehr als 300 bar ausgesetzt wird und man in den Träger ein Volumen an flüssigem Harz von mehr als wenigstens 2% des Volumens der offenen Poren eindringen und aushärten lässt, wobei die Volumina bei einer Temperatur von 20 °C und einem Druck von 1 bar gemessen sind. Durch das beschriebene Verfahren soll die Herstellung von Verbundblöcken ermöglicht werden, die sich durch eine hohe Festigkeit auszeichnen.

Die im Stand der Technik beschriebenen zahnärztlichen Werkstoffe weisen den Nachteil auf, dass sie in der Regel nach ihrer Bearbeitung einer Wärmebehandlung unterzogen werden müssen, um eine ausreichende Festigkeit zu erreichen, wobei die erforderlichen Temperaturen für diese Behandlung je nach Material bei bis zu 1600 °C liegen können. Damit sind solche Materialen für die sogenannte "Chair-side"-Behandlung ungeeignet, da die individuelle Anpassung und das letztendliche Einsetzen in mehreren Sitzungen vorgenommen werden müssen.

Die im Stand der Technik beschriebenen Verbundwerkstoffe weisen den Nachteil auf, dass es durch die Kombination mit dem Füllstoff meistens zu einer Abnahme der Festigkeit kommt, so dass diese Materialien nicht mehr für dentale Zwecke verwendbar sind.

Es ist daher die Aufgabe der vorliegenden Erfindung einen Werkstoff zur Verfügung zu stellen, der die für eine dentale Restauration nötige Festigkeit aufweist, sich leicht bearbeiten lässt und für die Chair-side-Behandlung geeignet ist, das heißt, dass zum Erreichen der nötigen Festigkeit nach der Bearbeitung und Anpassung der dentalen Restauration keine weitere Wärmebehandlung mehr nötig ist.

Es wurde überraschend gefunden, dass diese Aufgabe durch einen Rohling gelöst wird, der aus einer porösen Lithiumsilikatglaskeramik mit einer spezifischen Zusammensetzung und einem Füllstoff besteht.

Daher ist ein erster Gegenstand der vorliegenden Anmeldung ein Rohling bestehend aus einer porösen Lithiumsilikatglaskeramik und einem Füllstoff, wobei der Füllstoff die Poren der porösen Lithiumsilikatglaskeramik ganz oder teilweise ausfüllt, dadurch gekennzeichnet, dass die Lithiumsilikatglaskeramik aus einer Schmelze hergestellt wird, die wenigstens die folgenden Ausgangskomponenten umfasst:

| | |
|---|---|
| $SiO_2$: | 55 bis 70 Gew.-%, vorzugsweise 60 bis 65 Gew.-%; |
| $LiO_2$: | 10 bis 27 Gew.-%; vorzugsweise 11 bis 20 Gew.-%; |
| $ZrO_2$: | 5 bis 15 Gew.-%; vorzugsweise 7 bis 12 Gew.-%; |
| $Al_2O_3$: | 0,1 bis 5 Gew.-%; vorzugsweise 1,5 bis 4 Gew.-%; |
| $P_2O_5$: | 2 bis 10 Gew.-%; vorzugsweise 3 bis 7 Gew.-%; |

jeweils bezogen auf das Gesamtgewicht der Ausgangskomponenten.

Überraschenderweise hat sich gezeigt, dass durch die Kombination der hochfesten Glaskeramik mit dem Füllstoff ein hochfestes, elastisches Chair-side-Material für Einzelzahn- und Brückenversorgungen erhalten werden kann, das sich leicht bearbeiten lässt und nach der Bearbeitung keine weitere Wärmebehandlung erfordert, um die für eine dentale Restauration benötigte Festigkeit zu erreichen. Da der erfindungsgemäße Rohling bereits seine endgültige Festigkeit aufweist, kann er in jeder beliebigen Geometrie bereitgestellt werden, beispielsweise in Form eines Blocks, der dann direkt beim Zahnarzt bearbeitet und dem Patienten eingesetzt werden können.

Die Glaskeramik des erfindungsgemäßen Rohlings wird aus einer Glasschmelze hergestellt, die $SiO_2$, $Li_2O$, $ZrO_2$, $Al_2O_3$ und $P_2O_5$ enthält. Wie dem Fachmann bekannt, wird eine Glaskeramik dadurch hergestellt, dass die Ausgangskomponenten in einem ersten Schritt aufgeschmolzen werden und aus dieser Schmelze ein Glas gewonnen wird. Durch gezielte Kristallisation des Glases wird dann die Glaskeramik hergestellt. Da es während des Herstellungsprozesses zu Umwandlungen der ursprünglich eingesetzten Komponenten kommt, wird die Zusammensetzung der Glaskeramik in Bezug auf die ursprünglich eingesetzten Komponenten, als Ausgangskomponenten bezeichnet, angegeben.

Es wurde überraschend gefunden, dass durch die Kombination der Ausgangskomponenten in den angegebenen Mengen eine mechanische Festigkeit erreicht wird, die trotz der Porosität der Glaskeramik und der Anwesenheit des Füllstoffs in der Lage ist, den Kräften, die beispielsweise während des Kauens, auf die dentale Restauration einwirken, standzuhalten, so dass eine stabile und langlebige Restauration erhalten werden kann.

Zur Verbesserung der optischen Eigenschaften und zur Optimierung der Verarbeitbarkeit können der Schmelze, aus welcher die Glaskeramik hergestellt wird, weitere Komponenten hinzugefügt werden. So wurde gefunden, dass durch Zugabe von $CeO_2$ die Anzahl von Lufteinschlüssen in der Keramik deutlich reduziert werden konnte. Daher ist eine Ausführungsform bevorzugt, in der die Ausgangskomponenten der Glaskeramik weiterhin 0,5 bis 5 Gew.-%, vorzugsweise 1 bis 3,5 Gew.-% und alternativ 0,5 bis 2,5 Gew.-% $CeO_2$ umfassen, jeweils bezogen auf das Gesamtgewicht der Ausgangskomponenten.

In einer weiterhin bevorzugten Ausführungsform können die Ausgangskomponenten weitere Alkalioxide umfassen, insbesondere $Na_2O$ und $K_2O$. Dabei liegen die Mengen vorzugsweise bei 0 bis 4 Gew.-%, besonders bevorzugt bei 1 bis 3 Gew.-%, jeweils bezogen auf das Gewicht der Ausgangskomponenten. In einer besonders bevorzugten Ausführungsform umfassen die Ausgangskomponenten 0 bis 4 Gew.-%, besonders bevorzugt 1 bis 3 Gew.-% $K_2O$, jeweils bezogen auf das Gesamtgewicht der Ausgangskomponenten. Es wurde überraschend gefunden, dass durch die Zugabe von Kaliumoxid das Einschmelzverhalten der Mischung der Ausgangskomponenten positiv beeinflusst werden konnte und das erhaltene Glas eine verbesserte Verarbeitbarkeit aufwies.

In einer weiterhin bevorzugten Ausführungsform umfassen die Ausgangskomponenten zusätzlich $B_2O_3$, vorzugsweise in einer Menge von 0,5 bis 4 Gew.-%, besonders bevorzugt 1 bis 3 Gew.-%, jeweils bezogen auf das Gesamtgewicht der Ausgangskomponenten. Es wurde überraschend gefunden, dass durch die Zugabe von $B_2O_3$ zu der Mischung der Ausgangskomponenten die Erweichungstemperatur des Glases entsprechend den jeweiligen Anforderungen angepasst werden konnte. Weiterhin wurde ein positiver Effekt auf die Wasser-, Säure- und Laugenbeständigkeit beobachtet.

Um die optischen Eigenschaften der Glaskeramik anzupassen, können den Ausgangskomponenten weiterhin färbende Zusätze hinzugefügt werden, beispielsweise färbende Oxide. In einer bevorzugten Ausführungsform weisen die Ausgangskomponenten weiterhin $Tb_4O_7$ auf, vorzugsweise in einer Menge von 0,5 bis 3 Gew.-%, besonders bevorzugt 0,5 bis 2 Gew.-%, jeweils bezogen auf das Gesamtgewicht der Ausgangskomponenten.

Im Rahmen der vorliegenden Erfindung wurde überraschend gefunden, dass durch die Kombination einer Glaskeramik und eines Füllstoffs ein Werkstoff erhalten werden kann, der sich durch eine hohe Festigkeit bei gleichzeitig ausreichender Elastizität auszeichnet, um mittels abtragender Verfahren, beispielsweise CAD/CAM, bearbeitet zu werden, wobei selbst feine Strukturen ausgebildet werden können, ohne dass es zu einer übermäßigen Beanspruchung der Werkzeuge kommt. Um diese Kombination an Eigenschaften zu erreichen, hat es sich als vorteilhaft erwiesen, wenn der erfindungsgemäße Rohling einen Anteil an Lithiumsilikatglaskeramik aufweist, der 50 Vol.-% nicht unterschreitet. Daher ist eine Ausführungsform bevorzugt, in der der Anteil an Lithiumsilikatglaskeramik im erfindungsgemäßen Rohling 50 bis 99 Vol.-%, vorzugsweise 64 bis 95 Vol.-% beträgt, jeweils bezogen auf das Gesamtvolumen des Rohlings.

In diesem Zusammenhang ist ebenfalls eine Ausführungsform bevorzugt, in der der erfindungsgemäße Rohling einen Anteil von 1 bis 50 Vol.-%, vorzugsweise 5 bis 36 Vol.-% an Füllstoff aufweist, jeweils bezogen auf das Gesamtvolumen des Rohlings.

Dentale Restaurationen müssen eine hohe Festigkeit aufweisen, um den Kräften, die auf sie wirken, standhalten zu können. Diese Festigkeit zu erreichen, um beispielsweise eine Beschädigung der dentalen Restauration beim Kauvorgang zu vermeiden, ist insbesondere dann schwierig, wenn die dentale Restauration eine geringe Wandstärke aufweist und beispielsweise in Form eines Veneers vorliegt. Im Gegensatz dazu zeichnet sich der erfindungsgemäße Rohling insbesondere durch eine Kombination von mechanischen und elastischen Eigenschaften auf, die eine gute Bearbeitung des Rohlings zur Ausformung dentaler Restaurationen, wie beispielsweise Veneers, erlauben. Daher weist der Rohling in einer bevorzugten Ausführungsform eine Biegefestigkeit von 15 bis 300 MPa, vorzugsweise 70 bis 220 MPa auf, bestimmt mittels 3-Punktbiegefestigkeit. Eine Biegefestigkeit im beanspruchten Bereich macht den Rohling besonders für die Herstellung stabiler und langlebiger dentaler Restaurationen geeignet, auch bei dünner Wandstärke der Restauration. Im Rahmen der vorliegenden Erfindung hat sich überraschend gezeigt, dass entgegen den Beobachtungen im Zusammenhang mit Feldspatkeramiken, die Biegefestigkeit mit steigendem Keramikanteil nicht ab, sondern zunimmt, so dass eine vorteilhafte Kombination aus Festigkeit und Elastizität erzielt werden kann.

In einer weiterhin bevorzugten Ausführungsform weist der erfindungsgemäße Rohling ein Elastizitätsmodul von 10 bis 120 GPa, vorzugsweise 20 bis 100 GPa auf, bestimmt mittels 3-Punktbiegefestigkeit. Durch ein Elastizitätsmodul im beanspruchten Bereich kann eine werkzeugschonende Bearbeitung des Rohlings erreicht werden, wobei auch filigrane Strukturen und dünne Wandstärken bei den dentalen Restaurationen ausgearbeitet werden können, ohne dass es zur Ausbildung von Rissen oder Abplatzungen kommt.

Der erfindungsgemäße Rohling besteht aus einer porösen Glaskeramik, deren Poren zumindest teilweise, vorzugsweise vollständig, mit einem Füllstoff ausgefüllt sind. Hinsichtlich der Bearbeitbarkeit und Biokompatibilität des Rohlings hat es sich als vorteilhaft erwiesen, Kunststoffe als Füllstoff zu verwenden. Daher ist eine Ausführungsform bevorzugt, in der es sich bei dem Füllstoff um einen Kunststoff handelt, vorzugsweise um einen Kunststoff auf Basis von Poly(meth)acrylaten. In einer besonders bevorzugten Ausführungsform handelt es sich bei dem Füllstoff um einen Füllstoff auf Basis eines Kunststoffs, der aus Monomeren gebildet wird, die ausgewählt sind aus der Gruppe bestehend aus Urethandimethylacrylat, Triethylenglycoldimethacrylat und Mischungen hiervon.

Ein Nachteil, den die im Stand der Technik vorgeschlagenen Materialien für dentale Restauration aufweisen, liegt unter anderem darin, dass sie zwar eine hohe Festigkeit aufweisen, aber aufgrund dieser Festigkeit nur aufwendig zu bearbeiten sind und es insbesondere durch Rissbildung zu Beschädigungen der dentalen Restaurationen kommt. Um eine übermäßige Rissbildung zu vermeiden und eine gute Bearbeitbarkeit zu erreichen, hat es sich als vorteilhaft erwiesen, wenn der Rohling eine Risszähigkeit von mindestens 1 MPa*m$^{-0,5}$ aufweist. In einer bevorzugten Ausführungsform weist der erfindungsgemäße Rohling eine Risszähigkeit von 1 bis 2,2 MPa*m$^{-0,5}$. Die Risszähigkeit $K_{1C}$ wurde durch Ausmessen des Vickers-Härte-Ausdrucks mittels folgender Formel nach Munz/Fett (Munz D, Fett T. Ceramics - Mechanical Properties, Failure Behaviour, Materials Selection. Springer, 1999, 1st edition, p 36) bestimmt:

$$K_{IC} = 0,032 * H * \sqrt{a} * \left(\frac{E}{H}\right)^{\frac{1}{2}} * \left(\frac{c}{a}\right)^{-\frac{3}{2}}$$

$$H = \frac{F}{2a^2}$$

H: Härte in MPa

F: Eindrucklast in N

E: Elastizitätsmodul in MPa

a: halbe Eindruckdiagonale

c: halbe Rissdiagonale

Es wurde überraschend gefunden, dass der erfindungsgemäße Rohling eine hohe Festigkeit, ausgedrückt in Vickershärte, aufwies, wie sie insbesondere für dentale Anwendungen erforderlich ist. In einer bevorzugten Ausführungsform weist der Rohling eine Vickershärte [HV3] von 900 bis 7000 MPa, vorzugsweise 1000 bis 6000 MPa auf. Die Vickers Härte kann dabei nach DIN EN 843-4:2005 bestimmt werden.

Ein weiterer Gegenstand der vorliegenden Erfindung ist ein Verfahren zur Herstellung des erfindungsgemäßen Rohlings. Das Verfahren weist dabei die folgenden Schritte auf:

a) Bereitstellen eines Lithiumsilikatglases aus einer Schmelze hergestellt aus wenigstens den folgenden Ausgangskomponenten:

| | |
|---|---|
| $SiO_2$: | 55 bis 70 Gew.-%, vorzugsweise 60 bis 65 Gew.-%; |
| $LiO_2$: | 10 bis 27 Gew.-%; vorzugsweise 11 bis 20 Gew.-%; |
| $ZrO_2$: | 5 bis 15 Gew.-%; vorzugsweise 7 bis 12 Gew.-%; |
| $Al_2O_3$: | 0,1 bis 5 Gew.-%; vorzugsweise 1,5 bis 4 Gew.-% |
| $P_2O_5$: | 2 bis 10 Gew.-%; vorzugsweise 3 bis 7 Gew.-%; |

jeweils bezogen auf das Gesamtgewicht der Ausgangskomponenten;

b) Formgebung des Glases mittels Pressen oder Schlickerguss.

c) Sintern des Glases unter Erhalt einer porösen Lithiumsilikatglaskeramik, wobei der Sinterprozess beendet wird, bevor im Wesentlichen geschlossene Poren im Sinterprodukt entstehen;

d) Infiltrieren der in Schritt b) erhaltenen Lithiumsilikatglaskeramik mit einem Füllstoff;

e) Verfestigen des Füllstoffs.

In einer alternativen Ausführungsform des erfindungsgemäßen Herstellungsverfahrens wird der erfindungsgemäße Rohling dadurch gewonnen, dass aus der Schmelze der Ausgangskomponenten wie oben beschrieben ein Glas in Form eines Pulvers gewonnen wird, welches in einem anschließenden Schritt einem Kristallisationsprozess unterzogen wird. Das so gewonnene Glaskeramikpulver wird zu einem Vorrohling verpresst und unter Erhalt einer porösen Lithiumsilikatglaskeramik gesintert, wobei der Sinterprozess beendet wird, bevor im Wesentlichen geschlossene Poren im Sinterprodukt entstehen. Die so erhaltene poröse Lithiumsilikatglaskeramik wird dann wie oben beschrieben mit einem Füllstoff infiltriert und der Füllstoff ausgehärtet.

In einer bevorzugten Ausführungsform des erfindungsgemäßen Verfahrens weist das Sinterprodukt eine Porosität von 5 bis 85 Vol.-%, vorzugsweise 10 bis 50 Vol.-%, insbesondere 10 bis 35 Vol.-% auf, jeweils bezogen auf das Gesamtvolumen des Sinterprodukts. Die Porosität kann dabei beispielsweise mittels Quecksilberporosimetrie bestimmt werden.

In einer bevorzugten Ausführungsform wird das Sinterprodukt vor dem Infiltrieren mit dem Füllstoff einer Behandlung mit einem Kopplungsmittel unterzogen. Dadurch kann der Verbund zwischen der Lithiumsilikatglaskeramik und dem Füllstoff verbessert werden. Das Kopplungsmittel ist dabei vorzugsweise ausgewählt aus der Gruppe bestehend aus Aminopropyltriethoxysilan, Vinyltriethoxysilan, 3-Methyacryloxypropyl-trimethoxysilan oder eine Mischung derselben.

In einer bevorzugten Ausführungsform des erfindungsgemäßen Verfahrens wird das Sintern bei nicht mehr als 900 °C durchgeführt. In einer besonders bevorzugten Ausführungsform wird das Sintern bei einer Temperatur von 400 bis 800 °C durchgeführt. Es wurde überraschend gefunden, dass in diesem Temperaturbereich die Ausbildung von geschlossenen Poren vermieden werden kann. In einer besonders bevorzugten Ausführungsform wird das Sintern in einem zweistufigen Temperaturprozess durchgeführt, wobei die erste Temperatur vorzugsweise 400 bis 600 °C beträgt, während die Temperatur des sich anschließenden zweiten Sinterschritts vorzugsweise 600 bis 800 °C beträgt.

Der erfindungsgemäße Rohling zeichnet sich durch eine vorteilhafte Kombination an Eigenschaften aus, insbesondere hinsichtlich Festigkeit und Elastizität, die ihn besonders geeignet zur Herstellung von dentalen Restaurationen machen. Daher ist ein weiterer Gegenstand der vorliegenden Erfindung die Verwendung des erfindungsgemäßen Rohlings für dentale Zwecke, insbesondere zur Herstellung von Inlays, Onlays, Kronen, Brücken, Veneers, Abutments und Implantaten.

In einer besonders bevorzugten Ausführungsform wird der erfindungsgemäße Rohling zur Herstellung von Veneers, insbesondere von Veneers mit einer Wandstärke von weniger als 0,5 mm, vorzugsweise weniger als 0,3 mm verwendet.

Die vorliegende Erfindung wird anhand der folgenden Beispiele näher beschrieben, wobei diese keinesfalls als Einschränkung des Erfindungsgedanken zu verstehen sind.

Beispiele:

Es wurde eine Glasschmelze durch Mischen und anschließendes Aufschmelzen der folgenden Komponenten hergestellt, wobei die Angaben in Gew.-% sind, jeweils bezogen auf das Gesamtgewicht der Ausgangskomponenten:

Tabelle 1:

| Komponente | Gew.-% |
|---|---|
| $SiO_2$ | 60,0 |
| $Li_2O$ | 19,0 |
| $P_2O_5$ | 6,0 |
| $Al_2O_3$ | 2,0 |
| $K_2O$ | 2,0 |
| $ZrO_2$ | 10,3 |
| $CeO_2$ | 1,8 |
| $Tb_4O_7$ | 1,5 |
| $V_2O_5$ | 0,2 |
| $Y_2O_3$ | 0,5 |

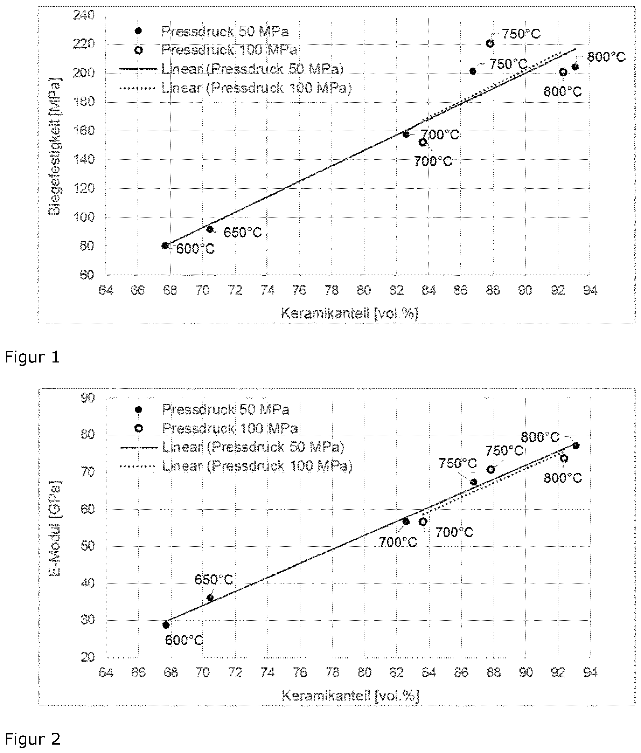

Die Schmelze wurde in kaltem Wasser abgeschreckt und zu einem Pulver gemahlen. Das anschließend unter Verwendung eines organischen Binders granulierte Material wurde mittels Presskraft auf eine Dichte von ca. 1,6 g/cm$^3$ gebracht. Die organischen Bestandteile des Granulats (Binder) wurden bei Temperaturen unterhalb 500 °C ausgebrannt. Alle Blöcke wurden zweistufig kristallisiert, wobei die erste Temperatur (Keimbildung) bei 550 °C lag und die zweite Temperatur (Verdichten und Kristallisieren) zwischen 600 und 800 °C. Die Kristallisationstemperaturen der jeweiligen Proben sind den Figuren 1 und 2 zu entnehmen.

Die porösen Blöcke wurden in einer 5%-igen Lösung von Methacryloxypropyltrimethoxysilan getränkt und getrocknet und dann über Nacht mit einer Mischung von einem Teil Urethandimethacrylat und einem Teil Triethylenglykoldimethylacrylat infiltriert und anschließend polymerisiert.

Die Eigenschaften der auf diese Weise hergestellten Proben sind in der folgenden Tabelle zusammengefasst:

Tabelle 2:

| Proben-Nr. | Pressdruck [MPa] | 2.Kristallisationstemperatur [°C] | Keramikanteil [Vol.-%] | Porosität [Vol.-%] | 3-Punkt-Biegefestigkeit [MPa] | E-Modul [GPa] | Vickers Härte HV3 [MPa] | Risszähigkeit K1C [MPa$\sqrt{m}$] |
|---|---|---|---|---|---|---|---|---|
| 1 | 50 | 600 | 67,7 | 32,3 | 80,4 | 28,8 | 1494 | 1,7 |
| 2 | 50 | 650 | 70,5 | 29,5 | 91,8 | 36,1 | 1882 | 1,5 |
| 3 | 50 | 700 | 82,6 | 17,4 | 157,7 | 56,7 | 3444 | 1,6 |
| 4 | 50 | 750 | 86,8 | 13,2 | 201,3 | 67,3 | 4507 | 1,7 |
| 5 | 50 | 800 | 93,1 | 6,9 | 204,5 | 77,2 | 5013 | 2,2 |
| 6 | 100 | 700 | 83,7 | 16,3 | 152,1 | 56,6 | 3579 | 1,5 |
| 7 | 100 | 750 | 87,8 | 12,2 | 220,5 | 70,7 | 4664 | 1,6 |
| 8 | 100 | 800 | 92,4 | 7,6 | 200,9 | 73,7 | 5092 | 1,8 |

Bei herkömmlichen Hybridkeramiken auf Basis von Feldspat wurde beobachtet, dass mit steigendem Gehalt an Füllstoff zwar die Bearbeitbarkeit zunahm, die Festigkeit aber abnahm. Im Gegensatz dazu wurde im Fall des erfindungsgemäßen Rohlings beobachtet, dass die Festigkeit wie auch das Elastizitätsmodul als Maß für die Bearbeitbarkeit mit steigendem Gehalt an Füllstoff ebenfalls zunahm, wie in den Figuren 1 und 2 zu erkennen.

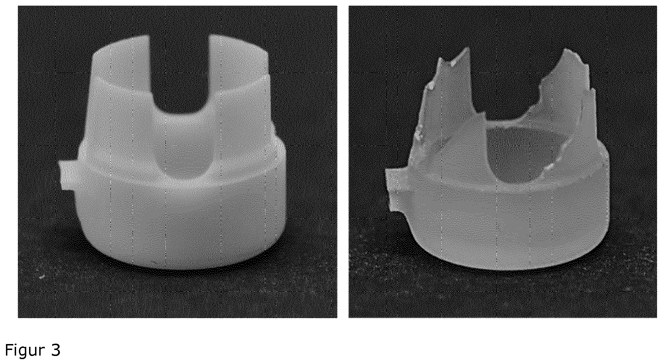

Die Bearbeitbarkeit mittels CAD/CAM-Verfahren wurde mittels internen Merlon-Tests bestimmt. Hierbei werden Probekörper geschliffen, die je 4 Zinnen aufweisen. Die Wandstärke der Zinnen variiert zwischen 0,2 und 0,5 mm. Je geringer die Wandstärke der schadensfrei herausgeschliffenen Probekörper desto besser die Bearbeitbarkeit mittels CAD/CAM-Verfahren. Die Probekörper sind in Figur 3 abgebildet, wobei beispielhaft links ein beschädigungsfrei herausgeschliffener Probekörper gemäß der vorliegenden Erfindung zu sehen ist und rechts ein Probekörper, bei dem die typischerweise auftretenden Beschädigungen zu sehen sind. Die folgende Tabelle zeigt die Ergebnisse zur CAD/CAM-Verarbeitbarkeit der Proben 2 bis 5 in Relation zu zwei Vergleichsmaterialien .

Tabelle 3:

| Material | Merlon | | | |
|---|---|---|---|---|
| Wandstärke | 0,2 mm | 0,3 mm | 0,4 mm | 0,5 mm |
| Glaskeramik (im vorkristallisierten Zustand) | 4 Zinnen leicht beschädigt | 1 Zinne leicht beschädigt | 1 Zinne leicht beschädigt | 1 Zinne leicht beschädigt |
| Polymerinfiltrierte Feldspatkeramik | keine Beschädigung | keine Beschädigung | keine Beschädigung | nicht durchgeführt |
| Pr. 2 | keine Beschädigung | keine Beschädigung | nicht durchgeführt | nicht durchgeführt |
| Pr. 3 | keine Beschädigung | keine Beschädigung | nicht durchgeführt | nicht durchgeführt |
| Pr. 4 | 1 Zinne leicht beschädigt | keine Beschädigung | nicht durchgeführt | nicht durchgeführt |
| Pr. 5 | 2 Zinnen leicht beschädigt | keine Beschädigung | nicht durchgeführt | nicht durchgeführt |

Wie der Tabelle zu entnehmen ist, weisen die erfindungsgemäßen Proben 2 bis 5 auch bei geringen Wandstärken eine gute Bearbeitbarkeit mittels CAD/CAM-Verfahren auf, was sie, gepaart mit der hohen Festigkeit, optimal für zur Herstellung von dentalen Restaurationen macht.

**Patentansprüche**

1. Rohling bestehend aus einer porösen Lithiumsilikatglaskeramik und einem Füllstoff, wobei der Füllstoff die Poren der porösen Lithiumsilikatglaskeramik ganz oder teilweise ausfüllt, **dadurch gekennzeichnet, dass** die Lithiumsilikatglaskeramik aus einer Schmelze hergestellt wird, die wenigstens die folgenden Ausgangskomponenten umfasst:

$SiO_2$: 55 bis 70 Gew.-%, vorzugsweise 60 bis 65 Gew.-%;
$LiO_2$: 10 bis 27 Gew.-%; vorzugsweise 11 bis 20 Gew.-%;
$ZrO_2$: 5 bis 15 Gew.-%; vorzugsweise 7 bis 12 Gew.-%;
$Al_2O_3$: 0,1 bis 5 Gew.-%; vorzugsweise 1,5 bis 4 Gew.-%
$P_2O_5$: 2 bis 10 Gew.-%; vorzugsweise 3 bis 7 Gew.-%;

jeweils bezogen auf das Gesamtgewicht der Ausgangskomponenten.

2. Rohling gemäß Anspruch 1, **dadurch gekennzeichnet, dass** der Rohling einen Anteil von 50 bis 99 Vol.-%, vorzugsweise 64 bis 95 Vol.-% an Lithiumsilikatglaskeramik aufweist, jeweils bezogen auf das Gesamtvolumen des Rohlings.

3. Rohling gemäß einem oder beiden der Ansprüche 1 und 2, **dadurch gekennzeichnet, dass** der Rohling einen Anteil von 1 bis 50 Vol.-%, vorzugsweise 5 bis 36 Vol.-% an Füllstoff aufweist, jeweils bezogen auf das Gesamtvolumen des Rohlings.

4. Rohling gemäß wenigstens einem der vorangehenden Ansprüche, **dadurch gekennzeichnet, dass** der Rohling eine Biegefestigkeit von 15 bis 300 MPa, vorzugsweise 70 bis 220 MPa aufweist, bestimmt gemäß 3-PunktBiegefestigkeit.

5. Rohling gemäß wenigstens einem der vorangehenden Ansprüche, **dadurch gekennzeichnet, dass** der Rohling ein Elastizitäts-Modul von 10 bis 120 GPa, vorzugsweise 20 bis 100 GPa aufweist, bestimmt gemäß 3-PunktBiegefestigkeit.

6. Rohling gemäß wenigstens einem der vorangehenden Ansprüche, **dadurch gekennzeichnet, dass** es sich bei dem Füllstoff um einen Kunststoff handelt, vorzugsweise einen Kunststoff auf Basis von Poly(meth)acrylat.

7. Rohling gemäß wenigstens einem der vorangehenden Ansprüche, **dadurch gekennzeichnet, dass** der Rohling eine Risszähigkeit von wenigstens 1 MPa*m$^{-0,5}$ vorzugsweise von 1 bis 2,2 MPa*m$^{-0,5}$, bestimmt nach Munz/Fett.

8. Verfahren zur Herstellung eines Rohling gemäß wenigstens einem der Ansprüche 1 bis 7, **dadurch gekennzeichnet, dass** das Verfahren die folgenden Schritte aufweist:

   a) Bereitstellen eines Lithiumsilikatglases aus einer Schmelze hergestellt aus wenigstens den folgenden Aus-gangskomponenten:

   $SiO_2$:  55 bis 70 Gew.-%, vorzugsweise 60 bis 65 Gew.-%;
   $LiO_2$:  10 bis 27 Gew.-%; vorzugsweise 11 bis 20 Gew.-%;
   $ZrO_2$:  5 bis 15 Gew.-%; vorzugsweise 7 bis 12 Gew.-%;
   $Al_2O_3$:  0,1 bis 5 Gew.-%; vorzugsweise 1,5 bis 4 Gew.-%
   $P_2O_5$:  2 bis 10 Gew.-%; vorzugsweise 3 bis 7 Gew.-% jeweils bezogen auf das Gesamtgewicht der Ausgangskomponenten;

   b) Formgebung des Glases mittels Pressen oder Schlickerguss.
   c) Sintern des Glases unter Erhalt einer porösen Lithiumsilikatglaskeramik, wobei der Sinterprozess beendet wird, bevor im Wesentlichen geschlossene Poren im Sinterprodukt entstehen;
   d) Infiltrieren der in Schritt c) erhaltenen Lithiumsilikatglaskeramik mit einem Füllstoff;
   e) Verfestigen des Füllstoffs.

9. Verfahren zur Herstellung eines Rohlings gemäß wenigstens einem der Ansprüche 1 bis 7, **dadurch gekennzeichnet, dass**

   a) aus einer Schmelze der Ausgangskomponenten ein Glas in Form eines Pulvers gewonnen wird, wobei die Ausgangskomponenten umfassen:

   $SiO_2$:  55 bis 70 Gew.-%, vorzugsweise 60 bis 65 Gew.-%;
   $LiO_2$:  10 bis 27 Gew.-%; vorzugsweise 11 bis 20 Gew.-%;
   $ZrO_2$:  5 bis 15 Gew.-%; vorzugsweise 7 bis 12 Gew.-%;
   $Al_2O_3$:  0,1 bis 5 Gew.-%; vorzugsweise 1,5 bis 4 Gew.-%

   $P_2O_5$: 2 bis 10 Gew.-%; vorzugsweise 3 bis 7 Gew.-%, jeweils bezogen auf das Gesamtgewicht der Ausgangskomponenten;
   b) Kristallisation des Pulvers unter Erhalt eines Lithiumsilikatglaskeramik-Pulvers;
   c) Verpressen des Pulvers zu einem Vorrohling;
   d) Sintern des gepressten Vorrohlings unter Erhalt einer porösen Lithiumsilikatglaskeramik, wobei der Sinterprozess beendet wird, bevor im Wesentlichen geschlossene Poren im Sinterprodukt entstehen;
   e) Infiltrieren der erhaltenen Lithiumsilikatglaskeramik mit einem Füllstoff;
   f) Verfestigen des Füllstoffs

10. Verfahren gemäß Anspruch 8 oder 9, **dadurch gekennzeichnet, dass** das Sinterprodukt eine Porosität von 5 bis 85 Vol.-%, vorzugsweise 10 bis 50 Vol.-%, insbesondere 10 bis 35 Vol.-% aufweist, jeweils bezogen auf das Gesamtvolumen des Sinterprodukts.

11. Verfahren gemäß einem oder mehreren der Ansprüche 8 bis 10, **dadurch gekennzeichnet, dass** das Sinterprodukt vor dem Infiltrieren einer Behandlung mit einem Kopplungsmittel unterzogen wird.

12. Verfahren gemäß Anspruch 11, **dadurch gekennzeichnet, dass** es sich bei den Kopplungsmittel um eines handelt, das ausgewählt ist aus der Gruppe bestehend aus Aminopropyltriethoxysilan, Vinyltriethoxysilan, 3-Methyacryloxypropyl-trimethoxysilan oder eine Mischung derselben.

13. Verfahren gemäß wenigstens einem der Ansprüche 8 bis 12, **dadurch gekennzeichnet, dass** das Sintern bei einer Temperatur von nicht mehr als 900 °C durchgeführt wird, vorzugsweise bei einer Temperatur von 400 bis 800 °C.

14. Verwendung eines Rohlings gemäß wenigstens einem der Ansprüche 1 bis 7 für dentale Zwecke, insbesondere für Inlays, Onlays, Kronen, Brücken, Veneers, Abutments und Implantaten.

15. Verwendung gemäß Anspruch 13, **dadurch gekennzeichnet, dass** der Rohling zur Herstellung eines Veneers, vorzugsweise eines Veneers mit einer Wandstärke von weniger als 0,5 mm, vorzugsweise weniger als 0,3 mm verwendet wird.

Figur 1

Figur 2

Figur 3

Europäisches
Patentamt

European
Patent Office

Office européen
des brevets

# EUROPÄISCHER RECHERCHENBERICHT

Nummer der Anmeldung

EP 19 19 1036

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2011/229858 A1 (SADOUN MICHAEL [FR]) 22. September 2011 (2011-09-22) * das ganze Dokument * * insbesondere Beispiele 1 bis 3 * ----- | 1-15 | INV. C03C10/00 C03C3/095 C03C14/00 A61K6/15 |
| Y | EP 0 803 241 A2 (G C DENTAL PRODUCTS CORP [JP]) 29. Oktober 1997 (1997-10-29) * Zusammenfassung * * Seite 3, Zeile 38 - Seite 8, Zeile 47 * * Abbildung 1 * * Ansprüche 1-9 * ----- | 1-15 | |
| Y | DE 10 2015 108171 A1 (DEGUDENT GMBH [DE]) 24. November 2016 (2016-11-24) * das ganze Dokument * * insbesondere [0001], [0018] bis [0021], [0027] * ----- | 1-15 | |

### RECHERCHIERTE SACHGEBIETE (IPC)

C03C
A61K

Der vorliegende Recherchenbericht wurde für alle Patentansprüche erstellt

| Recherchenort | Abschlußdatum der Recherche | Prüfer |
|---|---|---|
| München | 7. Februar 2020 | Heer, Stephan |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
   Dokument

EPO FORM 1503 03.82 (P04C03)

## ANHANG ZUM EUROPÄISCHEN RECHERCHENBERICHT
### ÜBER DIE EUROPÄISCHE PATENTANMELDUNG NR.

EP 19 19 1036

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-02-2020

| Im Recherchenbericht angeführtes Patentdokument | Datum der Veröffentlichung | Mitglied(er) der Patentfamilie | Datum der Veröffentlichung |
|---|---|---|---|
| US 2011229858 A1 | 22-09-2011 | CN 102149348 A | 10-08-2011 |
| | | EP 2331009 A1 | 15-06-2011 |
| | | ES 2633967 T3 | 26-09-2017 |
| | | FR 2935897 A1 | 19-03-2010 |
| | | JP 5276175 B2 | 28-08-2013 |
| | | JP 2012501783 A | 26-01-2012 |
| | | PL 2331009 T3 | 31-10-2017 |
| | | SI 2331009 T1 | 30-10-2017 |
| | | US 2011229858 A1 | 22-09-2011 |
| | | WO 2010029515 A1 | 18-03-2010 |
| EP 0803241 A2 | 29-10-1997 | EP 0803241 A2 | 29-10-1997 |
| | | US 5869548 A | 09-02-1999 |
| DE 102015108171 A1 | 24-11-2016 | AU 2016267543 A1 | 07-12-2017 |
| | | CA 2985148 A1 | 01-12-2016 |
| | | CN 107873020 A | 03-04-2018 |
| | | DE 102015108171 A1 | 24-11-2016 |
| | | EP 3095436 A1 | 23-11-2016 |
| | | JP 2018518442 A | 12-07-2018 |
| | | US 2016340239 A1 | 24-11-2016 |
| | | WO 2016188914 A1 | 01-12-2016 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014177659 A
- WO 2013167723 A
- EP 2269960 A

- WO 02076907 A
- US 20110229858 A

**In der Beschreibung aufgeführte Nicht-Patentliteratur**

- Ceramics - Mechanical Properties, Failure Behaviour. **MUNZ D ; FETT T.** Materials Selection. Springer, 1999, 36